**Europäisches Patentamt**

**European Patent Office**

**Office européen des brevets**

(19)

(11) Veröffentlichungsnummer : **0 359 097 B1**

(12) **EUROPÄISCHE PATENTSCHRIFT**

(45) Veröffentlichungstag der Patentschrift :
**12.08.92 Patentblatt 92/33**

(21) Anmeldenummer : **89116372.7**

(22) Anmeldetag : **05.09.89**

(51) Int. Cl.⁵ : **A61F 2/36,** A61F 2/46,
A61B 17/56

(54) **Hüftkopf-Schaft-Prothese.**

(30) Priorität : **06.09.88 DE 3830257**

(43) Veröffentlichungstag der Anmeldung :
**21.03.90 Patentblatt 90/12**

(45) Bekanntmachung des Hinweises auf die
Patenterteilung :
**12.08.92 Patentblatt 92/33**

(84) Benannte Vertragsstaaten :
**AT BE CH DE ES FR GB GR IT LI NL SE**

(56) Entgegenhaltungen :
**DE-A- 2 542 056**
**DE-A- 3 247 726**

(56) Entgegenhaltungen :
**DE-U- 8 605 099**
**FR-A- 2 501 998**
**GB-A- 2 197 204**
**US-A- 4 765 328**

(73) Patentinhaber : **Baumann, Friedrich**
**Am Kirchberg 2**
**W-8858 Neuburg/Donau (DE)**

(72) Erfinder : **Baumann, Friedrich**
**Am Kirchberg 2**
**W-8858 Neuburg/Donau (DE)**

(74) Vertreter : **Neubauer, Hans-Jürgen, Dipl.-Phys.**
**Fauststrasse 30**
**W-8070 Ingolstadt (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 359 097 B1

## Beschreibung

Die Erfindung betrifft eine Hüftkopf-Schaft-Prothese gemäß dem Oberbegriff des Anspruchs 1.

Eine bekannte Hüftkopf-Schaft-Prothese (FR-A-2501998) besteht aus einem länglichen Schaftteil für eine zementfreie Implantation im Markraum eines Oberschenkelhalsstumpfes und einem am Schaftteil anschließenden, gegenüber der Längsachse des Schaftteils seitlich abstehenden Hüftkopfteil. Der Schaft besteht aus einem Schaftoberteil und einem zylindrisch geformten Schaftunterteil, wobei der Schaftoberteil an der Lateralseite als gerade Verlängerung des zylindrischen Schaftunterteils ausgebildet ist und die gegenüberliegende Medialseite sich nach proximal konisch mit einem konkaven Bogen erweitert, so daß dadurch etwa eine Dreiecksfläche entsteht.

Die Dicke des Schaftoberteils bzw. der Dreiecksfläche ist gegenüber dem Durchmesser des zylindrischen Schaftunterteils kleiner. Dadurch ergeben sich zwischen dem Schaftoberteil und dem Markraum Spalte. Der fehlende, allseitige Formschluß und die dadurch möglichen Bewegungen des Prothesenschafts im Markraum haben eine negative Auswirkung auf die primäre Stabilität. Die Einheilung dauert relativ lange, da die Spalte durch neues Knochenwachstum weit überbrückt werden müssen. Zudem ist die Wirkung einer an sich bekannten Hydroxy-Apatitbeschichtung zur Induzierung der Knochenneubildung in den relativ weiten Spaltbereichen reduziert. Die neu gebildete Knochensubstanz, die nach der Einheilung die Spaltbereiche ausfüllt, ist im Vergleich zur angrenzenden Knochensubstanz eher weniger stabil, so daß die Gefahr einer Lockerung, insbesondere durch Mikrobewegungen, besteht.

Der Erfindung liegt das technische Problem zugrunde, eine Hüftkopf-Schaft-Prothese gemäß dem Oberbegriff des Patentanspruchs 1 so weiterzubilden, daß eine gute primäre Stabilität und gute Einheilung sowie hohe Langzeitstabilität erreicht wird.

Dieses Problem wird mit den kennzeichnenden Merkmalen des Anspruchs 1 gelöst.

Gemäß Anspruch 1 ist der Schaft mit einem Hydroxy-Apatitüberzug versehen und die Dreiecksfläche entspricht in der Dicke dem Durchmesser des zylindrischen Schaftunterteils.

Die Merkmale des Anspruchs 1 wirken bei der Lösung des technischen Problems in besonderem Maße kombinatorisch zusammen. Durch das zylindrisch geformte Schaftunterteil in Verbindung mit der entsprechenden Weiterführung in der Dreiecksfläche im Durchmesser des zylindrischen Schaftunterteils werden Umfangsflächen für einen Formschluß zwischen Schaft und Bohrung im Markraum geschaffen, wobei insbesondere auch der Schaft im Bereich seines Schaftoberteils mit seinem ganzen halben Umfang Flächenkontakt mit der Bohrung im Markraum hat. Damit wird in Verbindung mit dem Hydroxy-Apatitüberzug eine überraschend positive Wirkung hinsichtlich der primären Stabilität, des Einwachsens und der Langzeitstabilität erzielt.

Aufgrund der zylindrischen, durchgehenden Grundform des Schaftes ist eine Vorbereitung und ein Aufbohren des Markraums mit relativ einfachen Mitteln schnell durchführbar. Nach dem Implantieren der Hüftkopf-Schaft-Prothese sitzt diese durch den allumfassenden Formschluß fest, wobei sich kontinuierliche Kraftaufnahmeflächen ergeben und Radial- und Scherspannungen durch den gesamtflächigen Kontakt mit dem Knochen in annähernd gleicher Weise gering ohne punktuelle Extrembelastungen sind. Damit wird eine optimale primäre Stabilität erzielt.

Durch diese optimale primäre Stabilität werden Bewegungen und Mikrobewegungen des Schafts im Markraum vermieden, die die Einheilung stören und verzögern. Zusätzlich durch diesen, das Einwachsen begünstigenden Formschluß wird durch die große Kontaktfläche zwischen Knochen und Hydroxy-Apatitbeschichtung die bekannte osteopoetische Wirkung des Hydroxy-Apatits und damit des knöchernen Einbaus des Schaftteils gefördert, so daß insgesamt ein überraschend schnelles Einwachsen der Hüftkopf-Schaft-Prothese erzielt wird.

Durch den primären, engen Formschluß treten keine großen Spalte zwischen Schaft und Knochen auf, die durch neue und weniger feste Knochensubstanz überbrückt werden müßten, so daß dadurch verursachte Mikrobewegungen und örtliche Überbeanspruchungen des Knochens mit Sicherheit vermieden und auch Langzeitstabilität in hohem Maße erreicht wird.

Eine geeignete, gut implantierbare Form der Hüftkopf-Schaft-Prothese ergibt sich nach Anspruch 2 durch eine obere waagrechte Begrenzung der Dreiecksfläche, wobei der konkave Bogen gemäß Anspruch 3 etwa einen Tangentenneigungswinkel von 15° gegenüber der Längsachse hat und damit der physiologischen Krümmung des unteren Anteils des Schenkelhalses bis zum Trochanter minor weitgehend angepaßt ist.

Beim Einsetzen und bei Belastung des Beines wird der konische Schaftoberteil in den sich nach unten verjüngenden Knochen eingepreßt. Die parallel zur Kniegelenkstransversalachse verlaufende Dreiecksfläche verhindert eine Drehung des Prothesenschaftes im Oberschenkelmarkraum, wozu vorteilhaft auch der Schaftoberteil flächig und formschlüssig an dem entsprechend vorbereiteten und aufgebohrten Markraum anliegt. Zweckmäßig werden etwa zwei Drittel der Schaftlänge als Schaftunterteil zylindrisch ausgebildet.

Der Hüftkopfteil besteht nach Anspruch 4 aus einem an sich bekannten Konus, auf den ein Keramikkopf aufsetzbar ist. Dies beinhaltet vorteilhaft die Möglichkeit, verschiedene Schenkelhalslängen zu wählen, je nach der Tiefe der Bohrung des Keramikkopfes, in die der Konus dann mehr oder weniger tief eintritt.

Entsprechend den biomechanischen Anforderungen bildet nach Anspruch 5 die Konusachse in an sich bekannter Weise mit der Längsachse des Schaftteils einen Winkel von 40°.

Die Konusachse ist gegenüber der Dreiecksfläche gemäß Anspruch 6 um 10° nach vorne geneigt (Antetorsion). Damit ist beim Absägen des Schenkelhalses die Schnittebene in 90° zur Oberschenkelachse auszuführen und nicht wie bisher die Antetorsion bei der Schnittführung mit entsprechender Neigung zu berücksichtigen, was schwer kontrollierbar und schwierig exakt ausführbar ist.

Der Oberteil der Prothese ist kragenlos und sitzt am Schenkelhalsstumpf nach der Resektion nicht auf. Damit kann die nach der primären Stabilität gegebenenfalls eintretende Knochenresorbtion durch ein Nachsetzen ausgeglichen werden. Die zylindrische Aufbohrung des Markraums und die entsprechende Schaftform ermöglichen weiter ein press-fit bei gleichem Gesamtkontakt. Weil die Radial- und Scherspannungen durch diesen Gesamtkontakt relativ gering sind, kann eine Kragenabstützung entfallen. Mit Anspruch 7 werden aber dennoch zu beiden Seiten der Dreiecksfläche ventral und dorsal gegenüberliegend leistenförmige Kragen geringer Höhe vorgeschlagen. Diese ergeben nur einen geringen Aufsitz am knöchernen Schenkelhalsstumpf, ermöglichen aber den richtigen Sitz der Prothese zur Resektionsebene des Schenkelhalses zu überprüfen. Bevorzugt werden die leistenförmigen Kragen nach Anspruch 8 gegenüber der Längsachse in an sich bekannter Weise um 50° geneigt angebracht.

Nach Anspruch 9 ist es in an sich bekannter Art zweckmäßig, an einem lateralen, äußeren Bereich einen Werkzeugansatz zur verdrehsicheren Aufnahme eines Einschlaginstruments einzuformen. Der Werkzeugansatz kann beispielsweise als 6-Kant-Imbus-Bohrung ausgeführt sein.

Für eine schnelle und exakte Vorbereitung des Oberschenkelhalsmarkraums wird gemäß Anspruch 10 eine Vorfräse vorgeschlagen, die ein Führungsrohr mit einer daran angebrachten Auflagefläche zur Auflage auf der Resektionsebene und ein darin geführtes Frästeil mit Fräskopf umfaßt. Der Fräskopf soll nach Anspruch 11 scharfe Längsrippen aufweisen und stumpf zugespitzt sein. Zur Festlegung der Bohrtiefe ist am Werkzeugschaft ein Längsanschlag, z.B. in Form eines Ringes, angebracht. Die Anlagefläche steht in 90° zur Fräsenlängsachse und damit zur Oberschenkellängsachse. Damit ist bei fehlerhafter Schnittführung diese überprüfbar und korrigierbar durch eine auflageflächenparallele Nachsektion.

Nach der Verwendung der Vorfräse ist es erforderlich, den Markraum genau zylindrisch entsprechend dem zylindrischen Bereich des Prothesenschafts aufzubohren. Dazu wird mit Anspruch 12 eine Bohrbuchse vorgeschlagen, die aus einem Führungsrohr besteht, die ebenfalls eine Auflagefläche entsprechend der Anordnung an der Vorfräse aufweist. Ein Teil des Führungsrohrs soll dabei nach oben abstehen und ein Teil unterhalb der Auflagefläche in den Oberschenkelmarkraum einsteckbar sein. Durch die Bohrbuchse ist eine zylindrische Markraumfräse einführbar. Die schneidende Oberfläche der Fräse erstreckt sich über die gesamte Bohr- bzw. Fräslänge mit durch Querrillen unterbrochenen scharfen Längsrippen. Der Durchmesser der Fräse ist geringfügig größer als der gewählte Schaftdurchmesser. Die aufgebohrte Länge ist etwas größer als die gewählte Schaftlänge.

Für das schrittweise Auffräsen des Markraums stehen mit den verschiedenen Fräsenstärken korrespondierende Buchsen zur Verfügung, welche jeweils gegen die nächstgrößere ausgewechselt werden, bis zum endgültigen und nach der Röntgenausmessung festgelegten Durchmesser der Markraumauffräsung.

Als weiteres Instrument für eine schnelle und exakte Vorbereitung des Implantationsraumes wird mit Anspruch 13 eine Raspel vorgeschlagen, die einen unteren zylindrischen Führungsteil entsprechend dem zylindrischen Schaftunterteil der Prothese aufweist. Daran schließt sich ein Mittelteil mit scharfen Querrippen an, der die Form des entsprechenden Schaftoberteils der Prothese aufweist. Darauf folgt ein Oberteil mit einem Handgriff einer Schlagfläche und mit Querbohrungen. Die Schlagfläche dient zum Eintreiben mit dem Hammer und die Lochbohrungen dienen zum Einsetzen eines Stabs für das Zurück- und Ausschlagen.

Anstelle und alternativ zu der Bohrbuchse nach Anspruch 12 wird mit Anspruch 14 zum zylindrischen Aufbohren des Oberschenkelhalsstumpfes ein in den von Hand mit einer Vorfräse vorbereiteten Markraum einsetzbarer Führungsstab beansprucht. Dieser Führungsstab ist an seinem Ende für eine zentrische Abstützung im Markraum verbreitert. Zusammen mit dem Führungsstab ist eine zylindrische Markraumfräse als Hohlfräse zu verwenden, die über dem in seiner Länge aus dem Markraum ragenden Führungsstab gesteckt und somit zentrisch geführt wird.

Die Markraumfräse ist bevorzugt als Kronenfräse mit seitlich schneidenden Längsrippen ausgeführt.

Anhand von Ausführungsbeispielen wird die Erfindung mit weiteren Merkmalen, Einzelheiten und Vorteilen näher erläutert.

Es zeigen

Fig. 1 eine Seitenansicht einer Hüftkopf-Schaft-Prothese,

Fig. 2 eine um 90° verdrehte Ansicht der Hüftkopf-Schaft-Prothese nach Fig. 1,

Fig. 3 eine Ansicht des Hüftkopfteils entlang der Blickrichtung B aus Fig. 1,

Fig. 4 einen Schnitt entlang der Linie A-A aus Fig. 1,

Fig. 5 eine Ansicht einer Vorfräse,

Fig. 6 eine um 90° gedrehte Ansicht eines Führungsrohrs der Vorfräse nach Fig. 5,

Fig. 7 eine Bohrbuchse,

Fig. 8 eine um 90° verdrehte Ansicht der Bohrbuchse nach Fig. 7,

Fig. 9 eine Raspel,

Fig. 10 einen Schnitt durch einen Oberschenkelhalsstumpf mit einem eingesetzten Führungsstab und einer darüber geführten Markraumfräse als Hohlfräse.

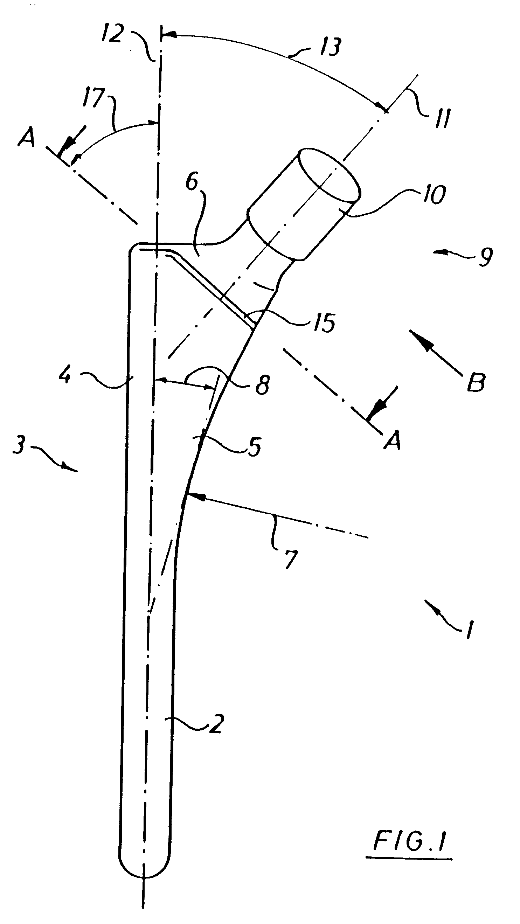

In Fig. 1 ist eine Hüftkopf-Schaft-Prothese 1 dargestellt, die aus einem unteren, zylindrisch geformten Schaftunterteil 2 und einem Schaftoberteil 3 besteht. Das Schaftoberteil 3 ist durch ein Dreieck gebildet, das durch eine gerade Verlängerung des Schaftunterteils 2 bildenden senkrechten Schenkel 4, einem konisch und konkav nach außen gebogenen Schenkel 5 und einem waagrechten Oberrand 6 begrenzt ist. Die Dreiecksfläche hat etwa die Dicke des zylindrischen Schaftunterteils 2.

Typische Abmessungen sind etwa eine Länge des Schaftunterteils und Schaftoberteils von 150 mm, ein zylindrischer Durchmesser des Schaftunterteils 2 von 12 mm, ein Radius 7 der konkaven Wölbung von 135 mm und ein Tangentialneigungswinkel 8 von 15°. Die Schaftlänge und der Durchmesser des zylindrischen Unterteils sind entsprechend der Röntgenausmessung in verschiedenen Größen, je nach den individuellen Maßen verfügbar.

Ein Hüftkopfteil 9 besteht aus einem Konus 10, dessen Konusachse 11 mit der Längsachse 12 des Prothesenschafts einen Winkel 13 von 40° einschließt.

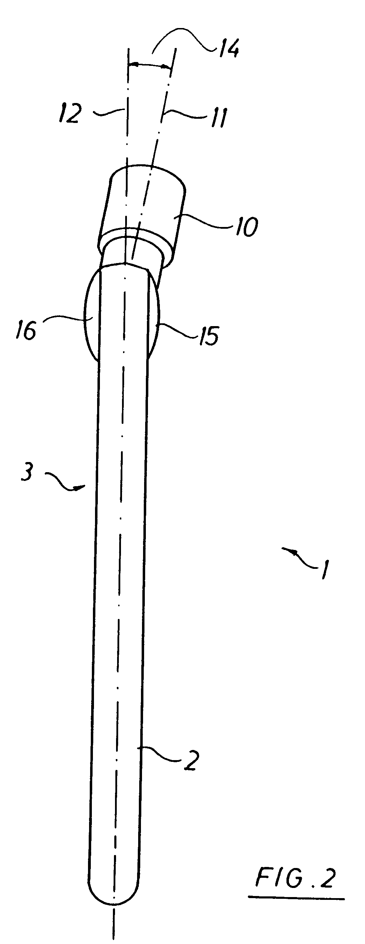

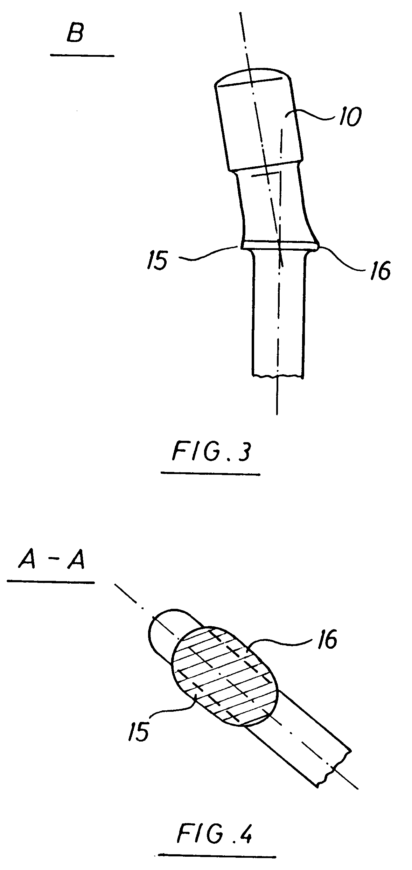

Der Konus 10 bzw. die Konusachse 11 ist zudem um einen Winkel 14 von 10° gegenüber der Längsachse 12 bzw. der Dreiecksfläche nach vorne geneigt, wie dies aus den Fig. 2 und 3 ersichtlich ist.

Zu beiden Seiten der Hüftkopf-Schaft-Prothese 1 sind gegenüberliegende, leistenförmige Kragen 15, 16 angeformt, die gegenüber der Längsachse 12 um einen Winkel 17 von 50° geneigt im Bereich des waagrechten Oberrandes 6 angebracht sind. Die Konusachse 11 und die Kragen 15, 16 stehen somit in der Ansicht der Fig. 1 senkrecht aufeinander.

In den Fig. 3 und 4 sind sowohl die Neigung (Antetorsion) des Konus 10 als auch die Lage und Ausdehnung der Kragen 15, 16 zu erkennen.

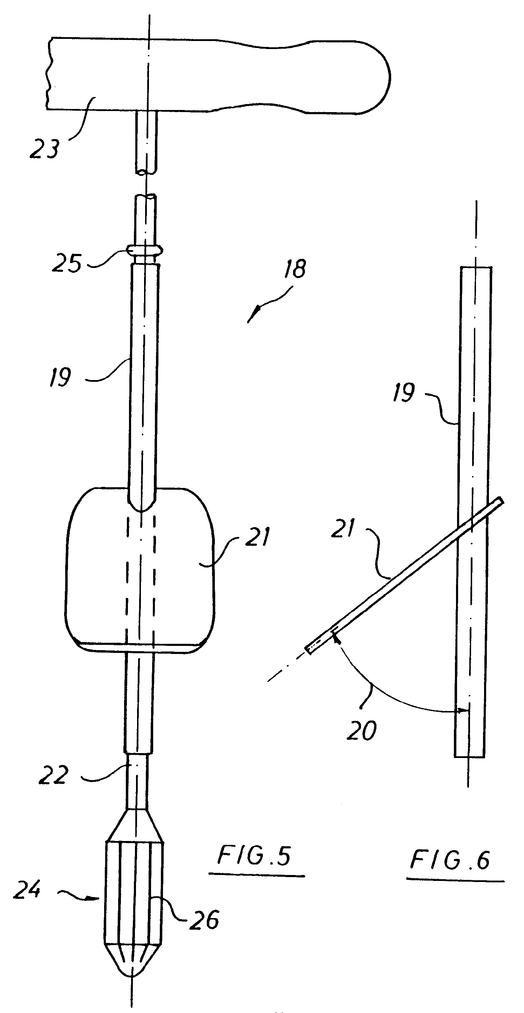

In Fig. 5 ist eine Vorfräse 18 dargestellt mit einem Führungsrohr 19, an dem in einem Winkel 20 von 50° eine Auflagefläche 21 angebracht ist. Im Führungsrohr 19 ist ein Werkzeugschaft 22 beweglich gehalten und geführt, der an der Oberseite einen Handgriff 23 trägt und an der Unterseite mit einem Fräskopf 24 ausgerüstet ist. Am Werkzeugschaft 22 ist ein Längsanschlag 25 in Form eines Ringes angebracht, der die Eindringtiefe des Fräskopfes 24 begrenzt. Der Fräskopf 24 enthält scharfe Längsrippen 26 und ist nach unten hin stumpf angespitzt.

In Fig. 6 ist das Führungsrohr 19 in einer um 90° gedrehten Stellung dargestellt zur Veranschaulichung des Neigungswinkels 20 der Auflagefläche 21.

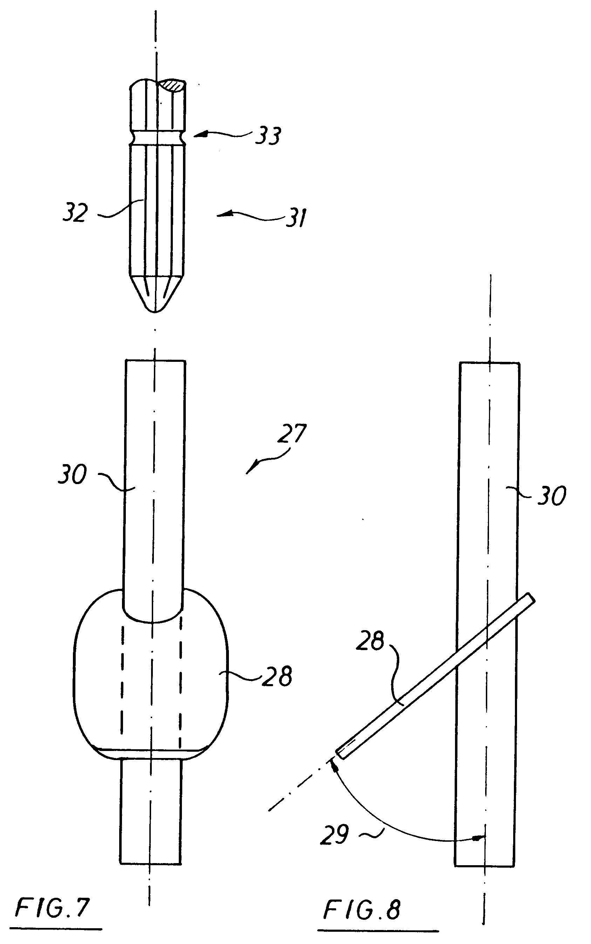

In den Fig. 7 und 8 ist eine Bohrbuchse 27 dargestellt, die ebenfalls eine etwa in ihrem mittleren Teil angebrachte Auflagefläche 28 mit einem Neigungswinkel 29 von 50° aufweist. Durch das Führungsrohr 30 der Bohrbuchse ist eine Markraumfräse 31 einführbar, die scharfe Längsrippen 32 und scharfe Quernuten 33 enthält.

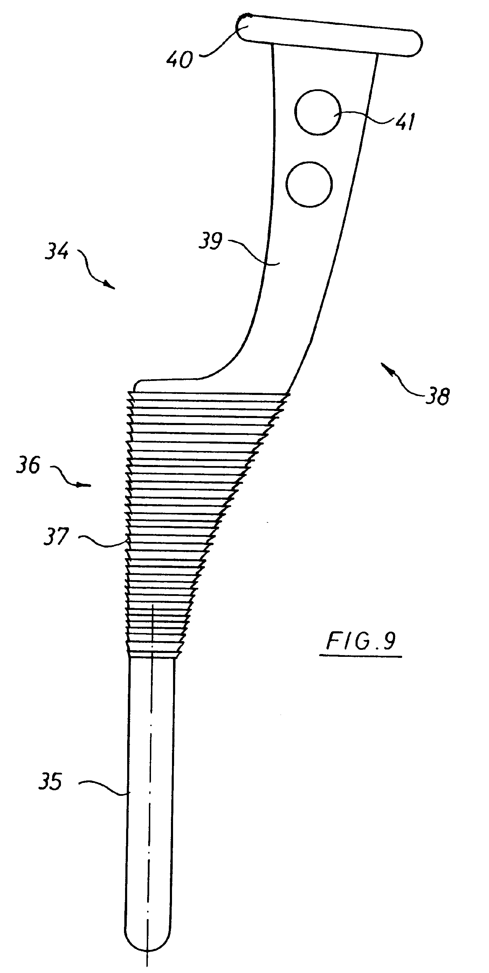

In Fig. 9 ist eine Raspel 34 gezeigt, mit einem unteren, zylindrischen Führungsteil 35 entsprechend dem zylindrischen Schaftunterteil der einzusetzenden Prothese. Ein Mittelteil 36 entspricht in seiner Form dem Schaftoberteil der Prothese und ist mit scharfen Querrippen 37 versehen. Ein Oberteil 38 ist als Griff 39 ausgebildet mit einer oberen Schlagfläche 40 und Querbohrungen 41.

Die Implantation der Hüftkopf-Schaft-Prothese wird folgendermaßen durchgeführt: Nach der Resektion des Hüftkopfs in dem mehrfach genannten Winkel von 50° wird die Vorfräse 18 aufgesetzt und der Markraum von Hand aufgebohrt. Der Fräskopf 24 hat dabei einen Durchmesser, der dem engsten Durchmesser des Markraums entspricht. Eine etwaige Fehlstellung der Schnittebene ist mit der Anlagefläche der Vorfräse erkennbar und planparallel zur Anlagefläche korrigierbar.

In einem nächsten Schritt wird mit Hilfe der Bohrbuchse 27 und der Markraumfräse 31 eine genaue zylindrische Bohrung hergestellt, wobei der Durchmesser der Markraumfräse dem Durchmesser des gewählten Prothesenschafts plus 0,5 mm entspricht. Die Länge der Markraumbohrung entspricht der einzuführenden Länge des Prothesenschafts plus 1 cm. Die Aufbohrung wird der Reihe nach mit immer größeren Werkzeugdurchmessern bis zur angegebenen Größe unter Verwendung der jeweils größeren Bohrbuchse durchgeführt.

Anschließend wird mit der Raspel 34 der Raum für den Oberteil 3 der Hüftkopf-Schaft-Prothese 1 vorbereitet. Die Raspel kann dabei über die Schlagfläche 40 mit einem Hammer eingetrieben und nach Durchstecken eines Stabes durch die Bohrungen 41 wieder herausgeschlagen werden.

EP 0 359 097 B1

Nach den vorstehenden Vorbereitungen wird die HüftkopfSchaft-Prothese 1 in den vorbereiteten Markraum eingeschlagen.

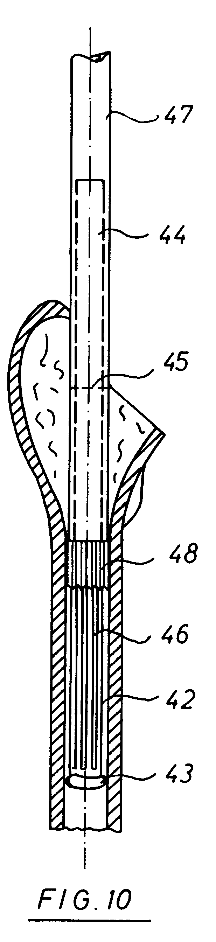

In Fig. 10 ist ein Querschnitt durch einen bereits mit einer Handfräse nach Fig. 5 vorbereiteten Oberschenkelhalsstumpf dargestellt, in den anstelle der Bohrbuchse nach Fig. 8 zum zylindrischen Aufbohren ein Führungsstab 42 eingesetzt ist. Der Führungsstab 42 ist an seinem unteren Ende durch einen Wulst 43 verbreitert, wobei sich dieser Wulst an den Seitenflächen des vorbereiteten Markraums abstützt und dort den Führungsstab 42 zentrisch im Markraum hält. Der Durchmesser des Führungsstabs ist dabei um etwa 1 bis 2 mm geringer als der Durchmesser des wulstförmigen bzw. abgeflacht kugelförmigen Endes.

Der Führungsstab 42 ragt mit seinem oberen Ende 44 etwa 6 bis 8 cm über die Schnittfläche des Schenkelhalsstumpfes heraus. Eine Markierung 45 gibt die Länge des einzuführenden Stabteils an, welche der Länge der zu implantierenden Schaftprothese plus einer Länge von etwa 10 mm entspricht.

Im Führungsstab sind Längsrillen 46 für das Ausbringen der gefrästen Teile vorgesehen.

Über das Führungsrohr ist eine (motorisch angetriebene) zylindrische Markraumfräse 47 gesteckt, die als Hohlfräse mit einem Innendurchmesser entsprechend dem Außendurchmesser des Führungsstabs 42 über den Führungsstab geführt wird. Die Markraumfräse 47 ist mit einem Fräskopf 48 als Kronenfräse mit seitlich schneidenden Längsrippen ausgeführt.

Die zylindrische Markraumfräse kann durch einen (nicht dargestellten) Anschlag am Führungsstab 42 nur auf eine bestimmte Länge (Länge der zu implantierenden Schaftprothese plus 10 mm) vorgetrieben werden.

Der Einsatz des beschriebenen Führungsrohrs 42 und der darüber geführten, zylindrischen Markraumfräse gewährleistet eine exakte, zentrische Führung im Markraum ohne Möglichkeit einer Seitenabweichung.

Die Ausfräsung des Markraums erfolgt auch bei der Verwendung des Führungsstabs 42 und der zylindrischen Markraumfräse 47 schrittweise unter wechselnder Anwendung der Hand-Vorfräsen und über jeweilig zugeordnete Führungsstäbe von Markraumfräsen bis zum Erreichen des vorgesehenen Durchmessers der zu implantierenden Schaftprothese.

Zusammenfassend wird festgestellt, daß mit der erfindungsgemäßen Hüftkopf-Schaft-Prothese eine gute primäre Stabilität und Langzeitstabilität bei schneller und guter Einheilung erreicht wird. Die richtige Stellung ergibt sich, wenn die genannte Dreiecksfläche parallel zur Transversalachse des Kniegelenks liegt, was operativ mit dem angegebenen Instrumentarium sicher erreichbar ist.

**Patentansprüche**

1. Hüftkopf-Schaft-Prothese
mit einem länglichen Shaftteil (2,3) für eine zementfreie Implantation im Markraum eines Oberschenkelhalsstumfes und
mit einem, am Schaftteil anschließenden, gegenüber der Längsachse (12) des Schaftteils (2,3) seitlich abstehenden Hüftkopfteil, wobei
der Schaftteil aus einem Schaftoberteil (3) und einem zylindrisch geformten Schaftunterteil (2) besteht und
der Schaftteil 2, im zylindrisch aufgebohrten Markraum implantierbar ist, wobei
der Schaftoberteil (3) an der Lateralseite als gerade Verlängerung des zylindrischen Schaftunterteils (2) ausgebildet ist und
die gegenüberliegende Medialseite sich nach proximal konisch mit einem konkaven Bogen (5) erweitert, so daß dadurch etwa eine Dreiecksfläche entsteht,
dadurch gekennzeichnet,
daß der Schaftteil (23) mit einem Hydroxy-Apatitüberzug versehen ist und
daß die Dreiecksfläche in der Dicke dem Durchmesser des zylindrischen Schaftunterteils (2) entspricht.
2. Hüftkopf-Schaft-Prothese nach Anspruch 1, dadurch gekennzeichnet, daß die Dreiecksfläche eine waagrechte Begrenzung (6) aufweist.
3. Hüftkopf-Schaft-Prothese nach Anspruch 2, dadurch gekennzeichnet, daß der konkave Bogen (5) etwa einen Tangentenneigungswinkel (8) von 15° gegenüber der Längsachse (12) aufweist und der physiologischen Krümmung des unteren Anteils des Schenkelhalses bis zum Trochanter minor weitgehend angepaßt ist.
4. Hüftkopf-Schaft-Prothese nach einem der Ansprüche 1 bis 3, dadurch gekennzeichnet, daß das Hüftkopfteil (9) aus einem Konus (10) besteht, auf den ein Keramikkopf aufsetzbar ist.
5. Hüftkopf-Schaft-Prothese nach Anspruch 4, dadurch gekennzeichnet, daß die Konusachse (11) mit der Längsachse (12) des Schaftteils (2) einen Winkel (13) von 40° bildet.
6. Hüftkopf-Schaft-Prothese nach Anspruch 4 oder 5, dadurch gekennzeichnet, daß die Konusachse (11) gegenüber der Dreiecksfläche um einen Winkel (14) von 10° nach vorne geneigt ist (Antetorsion).

5

7. Hüftkopf-Schaft-Prothese nach einem der Ansprüche 1 bis 6, dadurch gekennzeichnet, daß zu beiden Seiten der Dreiecksfläche, ventral und dorsal gegenüberliegend, leistenförmige Kragen (15, 16) geringer Höhe zur Überprüfung des richtigen Sitzes der Prothese bei der Implantation gegenüber der Resektionsebene des Schenkelhalses angeformt sind.

8. Hüftkopf-Schaft-Prothese nach Anspruch 7, dadurch gekennzeichnet, daß die leistenförmigen Kragen (15, 16) gegenüber der Längsachse (12) um 50° geneigt angebracht sind.

9. Hüftkopf-Schaft-Prothese nach einem der Ansprüche 1 bis 8, dadurch gekennzeichnet, daß an einem lateralen, äußeren Bereich ein Werkzeugansatz zur verdrehsicheren Aufnahme eines Einschlaginstruments eingeformt ist.

10. Hüftkopf-Schaft-Prothese nach Anspruch 1, gekennzeichnet durch

eine Vorfräse zum Aufbohren eines Oberschenkelhalsstumpfes für die Implantation der Hüftkopf-Schaft-Prothese, bestehend aus

einem Führungsrohr (19) mit einer dazu geneigt, bevorzugt im Winkel (20) von 50° gegenüber der Rohrachse, angebrachten Auflagefläche (21) zur Auflage auf der Resektionsebene und

einem Werkzeugschaft (22) mit größerer Länge als das Führungsrohr (19), der im Führungsrohr (19) beweglich gehalten und geführt ist, wobei an einer Endseite ein Handgriff (23) und an der Vorderseite ein zylindrischer Fräskopf (24) angebracht ist.

11. Hüftkopf-Schaft-Prothese nach Anspruch 10, dadurch gekennzeichnet, daß am Werkzeugschaft (22) der Vorfräse ein Längsanschlag (25) angebracht ist und der zylindrische Fräskopf (24) stumpf zugespitzt ist und scharfe Längsrippen (26) aufweist.

12. Hüftkopf-Schaft-Prothese nach Anspruch 1, gekennzeichnet durch

eine Bohrbuchse zum Aufbohren eines Oberschenkelhalsstumpfes für die Implantation der Hüftkopf-Schaft-Prothese,

wobei die Bohrbuchse (27) aus einem Führungsrohr (30) für eine Markraumfräse (31) besteht, mit einer dazu geneigt, bevorzugt im Winkel (29) von 50° gegenüber der Rohrachse, angebrachten Auflagefläche (28).

13. Hüftkopf-Schaft-Prothese nach Anspruch 1, gekennzeichnet durch

eine Raspel zur Vorbereitung eines Oberschenkelhalsstumpfes für die Implantation der Hüftkopf-Schaft-Prothese,

wobei die Raspel (34) aus einem unteren zylindrischen Führungsteil (35) entsprechend dem zylindrischen Schaftunterteil (2) der Prothese, einem mit scharfen Querrippen (37) versehenen Mittelteil (36) in der Form des entsprechenden Schaftoberteils (3) der Prothese und einem Oberteil (38) als Griff mit einer Schlagfläche (40) und Querbohrungen (41) besteht.

14. Hüftkopf-Schaft-Prothese nach Anspruch 1, gekennzeichnet durch

eine Fräse und Führung zum zylindrischen Aufbohren eines Oberschenkelhalsstumpfes für die Implantation der Hüftkopf-Schaft-Prothese,

wobei ein in den nach einem Vorfräsen von Hand vorbereite= ten Markraum einsetzbarer Führungsstab (42) vorgesehen ist und der Führungsstab (42) eine Endverbreiterung (43) zur zentrischen Abstützung im Markraum aufweist und wobei

eine zylindrische Markraumfräse (47) als Hohlfräse, die über dem in seiner Länge aus dem Markraum ragenden Führungsstab (42) einführbar ist, vorgesehen ist.

## Claims

1. A femoral head-shaft prosthesis with an elongate shaft part (2, 3) for a cement-free implantation in the medullary space on a femoral neck stump and

with a femoral head part following the shaft part and laterally projecting relative to the longitudinal axis (12) of the shaft part (2, 3), wherein

the shaft part consists of an upper shaft portion (3) and a cylindrically shaped lower shaft portion (2) and the shaft part (2, 3) can be implanted in the cylindrically drilled out medullary space, wherein

the upper shaft portion (3) is formed on the lateral side as a straight extension of the cylindrical lower shaft portion (2) and

the opposite medial side is flaring approximately conically with a concave arc (5) so that an approximately triangular area is obtained thereby,

characterized in that

the shaft part (2, 3) is provided with a hydroxyapatite coating and

that the triangular area corresponds in its thickness to the diameter of the cylindrical lower shaft portion (2).

2. A femoral head-shaft prosthesis according to claim 1, characterized in that the triangular area has a horizontal boundary (6).

3. A femoral head-shaft prosthesis according to claim 2, characterized in that the concave arc (5) has an approximately tangential angle of inclination (8) of 15° relative to the longitudinal axis (12) and is largely adapted to the physiological curvature of the lower portion of the femoral head as far as the lesser trochanter.

4. A femoral head-shaft prosthesis according to one of claims 1 to 3, characterized in that the femoral head part (9) consists on a cone (10), whereon there can be fitted a ceramic head.

5. A femoral head-shaft prosthesis according to claim 4, characterized in that the cone axis (11) norms an angle (13) on 40° with the longitudinal axis (12) on the shaft portion (2).

6. A femoral head-shaft prosthesis according to claim 4 or 5, characterized in that the cone axis (11) is forwardly inclined relative to the triangular area by an angle (14) on 10° (antetorsion).

7. A femoral head-shaft prosthesis according to one on claims 1 to 6, characterized in that on both sides on the triangular area, ventrally and dorsally opposed strip-shaped collars (15, 16) of small height are formed-on for checking the proper seating on the prosthesis relative to the resection plane on the femoral neck during the implantation.

8. A femoral head-shaft prosthesis according to claim 7, characterized in that the strip-shaped collars (15, 16) are arranged to slant by 50° relative to the longitudinal axis (12).

9. A femoral head-shaft prosthesis according to one of claims 1 to 8, characterized in that a tool accommodation point is formed-in in a lateral outer region to receive a driving-in implement, in a way secured against rotation.

10. A femoral head-shaft prosthesis according to claim 1, characterized by

a roughing cutter for drilling into a femoral neck stump for the implantation of the femoral head prosthesis, consisting of

a guide tube (19) with a bearing surface (21) slanting with respect thereto, being preferably arranged at an angle (20) of 50° relative to the tube axis for bearing on the resection plane and

a tool shank (22) with a longer length than the guide tube (19) which is movably held and guided in the guide tube (19), a handle (23) being arranged at the end and a cylindrical cutter head (24) at the front.

11. A femoral head-shaft prosthesis according to claim 10, characterized in that on the tool shank (22) of the roughing cutter, there is arranged a longitudinal stop (25) and the cylindrical cutter head (24) tapers to a blunt point and has sharp longitudinal ribs (26).

12. A femoral head-shaft prosthesis according to claim 1, characterized by

a drill sleeve for drilling into a femoral neck stump for the implantation of the femoral head-shaft prosthesis,

the drill sleeve (27) consisting of a guide tube (30) for a medullary space cutter (31) with a bearing surface (28) slanting with respect thereto, being preferably arranged at an angle (29) of 50° relative to the tube axis.

13. A femoral head-shaft prosthesis according to claim 1, characterized by

a rasp for preparing a femoral neck stump for the implantation of the femoral head-shaft prosthesis,

the rasp (34) consisting of a lower cylindrical guide part (35) corresponding to the cylindrical lower shaft portion (2) of the prosthesis, a central part (36) in the shape of the corresponding upper shaft portion (3) of the prosthesis, provided with sharp cross ribs (37), and an upper part (38) as a handle with an impact surface (40) and transverse holes (41).

14. A femoral head-shaft prosthesis according to claim 1, characterized by

a cutter and guide nor cylindrical drilling into a femoral neck stump for the implantation of the femoral head-shaft prosthesis,

wherein provision is made for a guide rod (42) that can be inserted into a medullary space prepared by hand after rough cutting, and the guide rod (42) has a widened end portion (43) for central support in the medullary space and wherein

provision is made for a cylindrical medullary space cutter (47) as a hollow cutter which can be inserted via the guide rod (42) projecting in its length from the medullary space.

**Revendications**

1. Prothèse de hanche à tige fémorale, comprenant une partie de tige allongée (2,3) en vue d'une implantation sans ciment dans le canal médullaire d'un tronçon de col du fémur,

et comprenant une partie de tête se raccordant à la partie de tige (2,3) et faisant latéralement saillie par rapport à l'axe longitudinal (12) de cette dernière, la partie de tige étant

constituée d'une partie de tige supérieure (3) et d'une partie de tige inférieure (2) de forme cylindrique, et la partie de tige (2,3) pouvant être implantée dans le canal médullaire creusé selon une forme cylindrique,

la partie de tige supérieure (3) étant configurée, sur le côté latéral, en prolongement rectiligne de la partie de tige inférieure cylindrique (2), et le côté médial opposé s'élargissant coniquement en direction proximale par un arc concave (5), de sorte qu'une surface approximativement triangulaire est formée,

caractérisée en ce que la partie de tige (2,3) est pourvue d'un revêtement d'hydroxy-apatite, et en ce que l'épaisseur de la surface triangulaire correspond au diamètre de la partie de tige inférieure cylindrique (2).

2. Prothèse de hanche à tige fémorale selon la revendication 1, carartérisée en ce que la surface triangulaire présente une délimitation horizontale (6).

3. Prothèse de hanche à tige fémorale selon la revendication 2, caractérisée en ce que l'arc concave (5) présente approximativement un angle d'inclinaison tangentiel (8) de 15° par rapport à l'axe longitudinal (12), et est largement adapté à la courbure physiologique de la partie inférieure du col du fémur, jusqu'au petit trochanter.

4. Prothèse de hanche à tige fémorale selon l'une des revendications 1 à 3, caractérisée en ce que la partie de tête (9) est constituée d'un cône (10) sur lequel peut être posée une tête en céramique.

5. Prothèse de hanche à tige fémorale selon la revendication 4, caractérisée en ce que l'axe (11) du cône forme un angle (13) de 40° avec l'axe longitudinal (12) de la partie de tige (2).

6. Prothèse de hanche à tige fémorale selon la revendication 4 ou 5, caractérisée en ce que l'axe (11) du cône est incliné vers l'avant d'un angle (14) de 10° par rapport à la surface triangulaire (torsion antérieure).

7. Prothèse de hanche à tige fémorale selon l'une des revendications 1 à 6, caractérisée en ce que des collets (15,16) en forme de rebords de faible hauteur, ventralement et dorsalement opposés, sont formés de part et d'autre de la surface triangulaire afin de contrôler, hors de l'implantation, l'assise correcte de la prothèse par rapport au plan de résection du col du fémur.

8. Prothèse de hanche à tige fémorale selon la revendication 7, caractérisée en ce que les collets en forme de rebords (15,16) sont disposés avec une inclinaison de 50° par rapport à l'axe longitudinal (12).

9. Prothèse de hanche à tige fémorale selon l'une des revendications 1 à 8, caractérisée en ce qu'un logement d'outil est formé sur une région extérieure latérale afin de recevoir de manière bloquée en rotation un instrument d'enfoncement de tige.

10. Prothèse de hanche à tige fémorale selon la revendication 1, caractérisée par une fraise ébaucheuse pour creuser un tronçon du col du fémur en vue de l'implantation de la prothèse de hanche à tige fémorale, la fraise ébaucheuse étant constituée

d'un tube de guidage (19) présentant une surface d'appui (21), à poser sur le plan de résection, qui est disposée sur le tube en étant inclinée par rapport à ce dernier, de préférence sous un angle (20) de 50° par rapport à l'axe du tube,

et d'une tige d'outil (22), qui est plus longue que le tube de guidage (19) et est maintenue et guidée en déplacement dans ce dernier, une poignée (23) étant disposée sur un côté terminal et une tête de fraise cylindrique (24) étant disposée sur le côté avant.

11. Prothèse de hanche à tige fémorale selon la revendication 10, caractérisée en ce qu'une butée longitudinale (25) est disposée sur la tige d'outil (22) de la fraise ébaucheuse, et la tête de fraise (24) est taillée en pointe émoussée et présente des nervures longitudinales prononcées (26).

12. Prothèse de hanche à tige fémorale selon la revendication 1, caractérisée par une douille de perçage pour creuser un tronçon du col du fémur en vue de l'implantation de la prothèse de hanche à tige fémorale, la douille de perçage (27) étant constituée d'un tube de guidage (30), destiné à guider une fraise (31) pour canal médullaire et présentant une surface d'appui (24) qui est disposée sur le tube en étant inclinée par rapport à ce dernier, de préférence sous un angle (29) de 50° par rapport à l'axe du tube.

13. Prothèse de hanche à tige fémorale selon la revendication 1, caractérisée par une râpe pour préparer un tronçon de col fémoral en vue de l'implantaison de la prothèse de hanche à tige fémorale,

la râpe (34) étant constituée d'une partie de guidage cylindrique inférieure (35) correspondant à la partie de tige inférieure cylindrique (2) de la prothèse, d'une partie centrale (36) dotée de nervures transversales prononcées (37) et présentant la forme de la partie de tige supérieure correspondante (3) de la prothèse, et d'une partie supérieure (34) comme poignée, avec une surface de frappe (40) et des trous transversaux (41).

14. Prothèse de hanche à tige fémorale selon la revendication 1, caractérisée par une fraise et un guide pour creuser sous une forme cylinrique un tronçon du col du fémur en vue de l'implantation de la prothèse de hanche à tige fémorale,

une barre de guidage (42) étant prévue, qui peut être introduite dans le canal médullaire préparé à la main par un fraisage ébaucheur, la barre de guidage (42) présentant une partie terminale élargie (43) en vue de son appui centré dans le canal médullaire,

et une fraise cylindrique pour canal médullaire (47) étant prévue sous la forme d'une fraise creuse, qui peut être enfilée sur la barre de guidage (42) dépassant longitudinalement hors du canal médullaire.

_FIG.1_

EP 0 359 097 B1

FIG.2

*B*

*FIG.3*

*A - A*

*FIG.4*

*FIG.5*

*FIG.6*

FIG.7

FIG.8

40

41

34

39

38

36

37

*FIG.9*

35

FIG. 10